# EUROPÄISCHE PATENTSCHRIFT

(11) **EP 1 984 304 B1**
(45) Veröffentlichungstag und Bekanntmachung des Hinweises auf die Patenterteilung: **05.05.2010**
(21) Anmeldenummer: 07704292.7
(22) Anmeldetag: 01.02.2007
(51) Int. Cl.: C02F 1/72, A61L 9/01, C02F 3/34

(54) **VERWENDUNG VON NITRATSALZEN ZUR UNTERDRÜCKUNG STÖRENDER GERÜCHE**
USE OF NITRATE SALTS FOR SUPPRESSING DISTURBING ODOURS
UTILISATION DE SELS DE NITRATE POUR LA SUPPRESSION DE MAUVAISES ODEURS

(30) Priorität: 08.02.2006 DE 102006006016
(43) Veröffentlichungstag der Anmeldung: 29.10.2008
(73) Patentinhaber: Sachtleben Chemie GmbH, 47198 Duisburg (DE)
(72) Erfinder: VOLLMUTH, Stefan, 47803 Krefeld (DE)
(74) Vertreter: Uppena, Franz
(86) Internationale Anmeldenummer: PCT/EP2007/050970
(87) Internationale Veröffentlichungsnummer: WO 2007/090782

(56) Entgegenhaltungen:
- WO-A-03/068692
- WO-A-2004/108173
- US-A- 4 911 843
- POPE R J: "COLLECTION SYSTEM ODORS - GOING WITH THE FLOW" AIR & WASTE MANAGEMENT, ANNUAL MEETING & EXHIBITION, Bd. 89, 23. Juni 1996 (1996-06-23), Seiten 1-12, XP008026461

## Beschreibung

Gegenstand der vorliegenden Erfindung ist die Verwendung von Nitratsalzen zur Unterdrückung störender biologisch erzeugter Gerüche im Sanitärbereich.

Dabei sind die Begriffe "Unterdrückung" bzw. "unterdrücken" im Sinne der Begriffe "Vermeidung und/oder Beseitigung" bzw. "vermeiden und/oder beseitigen" zu verstehen.

Insbesondere behandelt die vorliegende Erfindung die Verwendung von Nitratsalzen, um biologische Geruchsentwicklung im Sanitärbereich, insbesondere in Abflüssen, Ausgüssen, Siphons, Hebeanlagen und/oder in Abwasser führenden Bereichen zu unterdrücken. Als Nitratsalze werden Aluminiumnitrat, Aluminiumnitratsulfat, Calciumnitrat, Eisennitrat oder Mischung aus ein oder mehreren dieser Stoffe eingesetzt.

In vielen Haushalten, aber auch in öffentlichen Gebäuden oder Industriekomplexen kommt es in Abflüssen, Ausgüssen, Siphons, Hebeanlagen und/oder in Abwasser führenden Bereichen zu unangenehmer Geruchsbildung. Diese Gerüche rühren in der Regel von anaeroben biologischen Prozessen in den entsprechenden Systemen her. Zumeist handelt es sich bei den Geruch erzeugenden Stoffen um organische Sulfide oder Amine, aber auch um Schwefelwasserstoff. Letzterer ist ein hochtoxisches Gas mit dem sehr unangenehmen Geruch von faulen Eiern.

Traditionell wird eine solche Geruchsbildung beseitigt, indem der in den Abwasserleitungen befindliche Biofilm durch Desinfektion mit Chlorbleichlauge, Natronlauge und/oder Wasserstoffperoxid zerstört wird. Diese Chemikalien sind in ihren Eigenschaften ätzend, reizend und oxidierend und können bei nicht sachgemäßer Anwendung zu schweren Verletzungen führen.

Unter anaeroben Bedingungen wird im Wasser gelöstes Sulfat durch Sulfat reduzierende Bakterien reduziert, wodurch Schwefelwasserstoff entsteht. Der Schwefel des Sulfats dient den Bakterien dabei als Elektronenakzeptor. Dieser Prozess findet jedoch erst statt, wenn die Sauerstoffsättigung unter 20 % fällt. Der anaerobe biologische Abbau von Nitrat beginnt dem gegenüber schon, wenn die Sauerstoffsättigung unter 80 % fällt, so dass für bestimmte Bakterien der Stickstoff des Nitrats leichter und schneller als Elektronenakzeptor zur Verfügung steht als der Schwefel des Sulfats. Die Anwesenheit geringer Mengen Nitrat unterdrückt daher die biogene Sulfatreduktion und damit die Schwefelwasserstoffbildung. Darüber hinaus werden bei Anwesenheit von Nitrat vor allem Nitrat reduzierende Bakterien in den Biofilmen gegenüber Sulftat reduzierenden Bakterien bevorzugt. Dies führt zu einer Zunahme der Nitrat reduzierenden Bakterien und einer Abnahme der Sulfat reduzierenden Bakterien im Biofilm. Dieser Umstand wird seit Jahren in Abwasserkanalnetzen sowie geschlossenen Systemen zur Wasseraufbereitung, beispielsweise in Autowaschstrassen, sehr erfolgreich genutzt, um mit Calciumnitrat und nitrathaltigen Aluminiumsalzen die Bildung biogenen Schwefelwasserstoffs zu unterdrücken.

Aus EP-A-559272 ist weiterhin ein Verfahren bekannt, bei dem Nitrat zur Verminderung von Schwefelwasserstoff und anderen Geruchsbestandteilen in Jauche verwendet wird. Grundlage dieses Verfahrens ist jedoch nicht eine biologische Reaktion, sondern eine chemische Oxidation.

Aus der WO 2004/108173 ist ein Verfahren bekannt, bei dem Nitratsalze zur Unterdrückung biologisch erzeugter Gerüche verwendet werden.

Bekannt sind außerdem Rohrreiniger, die neben Alkalihydroxiden und Aluminium auch Nitratsalze enthalten. Ein Beispiel für solch einen Rohrreiniger ist Drano^{®} Rohrfrei der Firma Johnson Wax GmbH.

Jedoch wird in diesem Rohrreiniger Nitrat zur Oxidation und damit zur Vermeidung von explosivem Wasserstoff eingesetzt, der sich durch die Umsetzung von Aluminium mit Wasser bildet. Das Nitrat hat keine Wirkung auf den eigentlichen Reinigungsprozess und dient nicht der Unterdrückung störender biologisch erzeugter Gerüche.

Eine der großtechnischen Lösung vergleichbare Verwendung von Nitratsalzen zur Unterdrückung störender biologisch erzeugter Gerüche in Abwasser führenden Bereichen steht für Kleinstanwendungen, beispielsweise in Haushalt, Gebäudereinigung und Gebäudemanagement, bisher nicht zur Verfügung.

Aufgabe der vorliegenden Erfindung ist es daher, ein Verfahren bereitzustellen, das störende biologisch erzeugte Gerüche im Sanitärbereich, insbesondere in Abflüssen, Ausgüssen, Siphons, Hebeanlagen und/oder in Abwasser führenden Bereichen unterdrückt und problemlos in Kleinstanwendungen, beispielsweise in Haushalt, Gebäudereinigung und Gebäudemanagement, eingesetzt werden kann.

Erfindungsgemäß wird dieses Problem überraschenderweise durch die Merkmale des Hauptanspruchs gelöst. Vorzugsweise Ausgestaltungen finden sich in den Unteransprüchen. Insbesondere wird die Aufgabe durch die erfindungsgemäße Verwendung von nitrathaltigen Mischungen zur Unterdrückung störender biologisch erzeugter Gerüche gelöst.

Nach der erfindungsgemäßen Verwendung werden dabei Nitratsalze und/oder deren wässrige Lösungen in handelsübliche Haushaltsreiniger als Additiv zugegeben.

Hierbei ersehen Aluminiumnitrat, Aluminiumnitratsulfat und/oder Eisennitrat, und/oder deren wässrige Lösungen üblicherweise in Reinigungsmitteln eingesetzte Säurekomponenten, beispielsweise Zitronensäure oder Essigsäure.

Durch die regelmäßige Anwendung der Reinigungsmittel und deren Ableiten mit dem Abwasser in Ausgüsse und Abwasserleitungen steht den Biofilmen immer eine ausreichende Menge Nitrat zur Verfügung, so dass die Geruch bildenden Prozesse unterdrückt werden.

In einer weiteren erfindungsgemäßen Verwendung können die Nitratsalze oder deren wässrige Lösungen auch vorbeugend in geringen aber regelmäßigen Dosierungen in Abflüsse eine Geruchsbildung verhindern. Mischungen mit Biodispergatoren wie beispielsweise Orangenterpenen führen gleichzeitig zu einer Vermeidung von Biofilmaufwuchs an den Rohrwandungen. Mischungen mit speziellen Bakterienkulturen führen zu einer Beeinflussung des Biofilms, wodurch Nitrat reduzierende Bakterien sich schneller im Biofilm etablieren können.

Erfindungsgemäß kann für alle beschriebenen erfindungsgemäßen Verwendungen das von der Sachtleben Chemie GmbH unter der Marke N ICASAL vertriebene Polyaluminiumnitratsulfat als nitrathaltige Mischung eingesetzt werden. Dieses Polyaluminiumnitratsulfat ist in EP-A-1108679 bzw. EP-B-1108679, deren Offenbarungen vollumfänglich Teil dieser Beschreibung sind, offenbart und enthält 5,0 bis 5,6 Gew.-% Aluminium (Al³⁺), 2,4 bis 3,2 Gew.-% Sulfat und 14,5 bis 25,0 Gew.-% Nitrat. Das Polyaluminiumnitratsulfat kann sowohl als Additiv zu handelsüblichen Reinigungsmitteln zugegeben werden, als auch als Ersatz von Säurekomponenten, beispielsweise Zitronensäure oder Essigsäure dienen, als auch unmittelbar zur Unterdrückung störender biologisch erzeugter Gerüche eingesetzt werden.

Gegenstand der vorliegenden Erfindung ist im Einzelnen:
- die Verwendung von Nitratsalzen zur Unterdrückung störender biologisch erzeugter Gerüche im Sanitärbereich, insbesondere in Abflüssen, Ausgüssen, Siphons, Hebeanlagen und/oder in Abwasser führenden Bereichen, durch Unterdrückung der biogenen Sulfatreduktion;
- die erfindungsgemäße Verwendung von Nitratsalzen, wobei die Nitratsalze als Additive zu Reinigungsmitteln, vorzugsweise handelsüblichen Reinigungsmitteln, zugegeben werden;
- die erfindungsgemäße Verwendung von Nitratsalzen, wobei die Nitratsalze, Aluminiumnitrat, Aluminiumnitratsulfat, Eisennitrat oder Mischungen aus diesen, als Ersatz von organischen Säuren, vorzugsweise Zitronensäure und/oder Essigsäure, und/oder deren Salzen in Reinigungsmitteln, vorzugsweise handelsüblichen Reinigungsmitteln, eingesetzt werden;
- die erfindungsgemäße Verwendung Nitratsalzen, wobei die Nitratsalze in Form von wässrigen Lösungen eingesetzt werden;
- die erfindungsgemäße Verwendung von Nitratsalzen, wobei die Konzentration der Nitrat-lonen in den wässrigen Lösungen 0,5 bis 50, bevorzugt 5 bis 40, besonders bevorzugt 10 bis 35 Gew.-% beträgt;
- die erfindungsgemäße Verwendung von Nitratsalzen, wobei 70 bis 90 Gew.-% eines Reinigungsmittels mit 10 bis 30 Gew.-% einer wässrigen Aluminiumnitrat-Lösung gemischt werden;
- die erfindungsgemäße Verwendung von Nitratsalzen, wobei die Aluminiumnitrat-Lösung 2 bis 6 Gew.-% Al³⁺ und 20 bis 40, bevorzugt 25 bis 35 Gew.-% NO₃⁻ enthält;
- die erfindungsgemäße Verwendung von Nitratsalzen, wobei 70 bis 90 Gew.-% eines Reinigungsmittels mit 10 bis 30 Gew.-% einer wässrigen Aluminiumnitratsulfat-Lösung gemischt werden;
- die erfindungsgemäße Verwendung von Nitratsalzen, wobei die Aluminiumnitratsulfat-Lösung 4 bis 7 Gew.-% Al³⁺, 12 bis 24 Gew.-% NO₃⁻ und 2 bis 3,5 % Gew.-SO₄²⁻ enthält;
- die erfindungsgemäße Verwendung von Nitratsalzen, wobei die nitratsalzhaltigen Lösungen 0,01 bis 5, bevorzugt 0,1 bis 5, besonders bevorzugt 0,5 bis 2 Gew.-% mindestens eines Biodispergators, bevorzugt Orangenterpen, enthalten;
- die erfindungsgemäße Verwendung von Nitratsalzen, wobei die Mischungen von nitratsalzhaltigen Lösungen mit Reinigungsmitteln 0,01 bis 5, bevorzugt 0,1 bis 5, besonders bevorzugt 0,5 bis 2 Gew.-% mindestens eines Biodispergators, bevorzugt Orangenterpen, enthalten;
- die erfindungsgemäße Verwendung von Nitratsalzen, wobei die nitratsalzhaltigen Lösungen 0,01 bis 5, bevorzugt 0,05 bis 2, besonders bevorzugt 0,1 bis 1 Gew.-% einer wässrigen Suspension aus Schadstoff abbauenden Bakterien enthalten;
- die erfindungsgemäße Verwendung von Nitratsalzen, wobei die Mischungen von nitratsalzhaltigen Lösungen mit Reinigungsmitteln 0,01 bis 5, bevorzugt 0,05 bis 2, besonders bevorzugt 0,1 bis 1 Gew.-% einer wässrigen Suspension aus Schadstoff abbauenden Bakterien enthalten;

Die Erfindung wird durch die nachstehenden Beispiele näher erläutert, ohne sie dadurch einzuschränken:

### Beispiel 1a

Handelsüblicher Badreiniger bestehend aus 5,5 Gew.-% Zitronensäure, 0,3 Gew.-% Duftstoffe, 0,5 Gew.-% Fettalkoholpolyglykolether, 0,6 Gew.% Alkylpolyglucosid, 0,5 Gew.-% Etoxyalkohol und 0,23 Gew.-% Kaliumtripolyphosphat und 92,37 Gew.-% Wasser.

### Beispiel 1 b

Es werden 80 Gew.-% eines handelsüblichen Badreinigers nach Beispiel 1a mit 20 Gew.-% einer wässrigen Aluminiumnitratsulfat-Lösung bestehend aus 5,35 Gew.-% Al³⁺, 16,0 Gew.-% NO₃⁻ und 2,8 Gew.-% SO₄²⁻ gemischt. Das Produkt ist stabil und zeigt die vergleichbare Reinigungsleistung wie Beispiel 1 a.

### Beispiel 1c

Mischung auf Basis des handelsüblichen Badreinigers nach Beispiel 1a in dem die Zitronensäure und das Wasser durch eine wässrige Aluminiumnitratsulfat-Lösung bestehend aus 5,35 Gew.-% Al³⁺, 16,0 Gew.-% NO₃⁻ und 2,8 Gew.-% SO₄²⁻ ersetzt werden.
Die Zusammensetzung enthält: 0,3 Gew.-% Duftstoffe, 0,5 Gew.-% Fettalkoholpolyglykolether, 0,6 Gew.-% Alkylpolyglucosid, 0,5 Gew.-% Etoxyalkohol, 0,23 Gew.-% Kaliumtripolyphosphat, 5,24 Gew.-% Al³⁺, 15,7 Gew.-% NO₃⁻, 2,7 Gew.-% SO₄²⁻ und 74,23 Gew.-% Wasser. Das Produkt ist stabil und zeigt die vergleichbare Reinigungsleistung wie Beispiel 1 a.

### Beispiel 2a

Handelsüblicher WC-Reiniger bestehend aus 5,5 Gew.-% Zitronensäure, 1,0 Gew.-% Natriumalkylsulfonat (60%), 0,3 Gew.-% Duftstoffe, 0,002 Gew.-% Farbstoff, 0,6 Gew.-% Xanthan und 92,6 Gew.-% Wasser.

### Beispiel 2b

Es werden ein handelsüblicher WC-Reiniger nach Beispiel 2a mit 20 Gew.% einer wässrigen Aluminiumnitratsulfat-Lösung bestehend aus 5,35 Gew.% Al³⁺, 16,0 Gew.-% NO₃⁻ und 2,8 Gew.-% SO₄²⁻ gemischt. Das Produkt ist stabil und zeigt die vergleichbare Reinigungsleistung wie Beispiel 2a.

### Beispiel 2c

Mischung auf Basis des handelsüblichen Badreinigers nach Beispiel 2a in dem 46,3 Gew.-% Wasser durch eine wässrigen Aluminiumnitratsulfat-Lösung bestehend aus 5,35 Gew.-% Al³⁺, 16,0 Gew.-% NO₃⁻ und 2,8 Gew.-% SO₄²⁻ ersetzt werden. Zusammensetzung: 1,0 Gew.-% Natriumalkylsulfonat (60%), 0,3 Gew.-% Duftstoffe, 0,002 Gew.-% Farbstoff, 0,6 Gew.-% Xanthan, 2,87 Gew.-% Al³⁺, 8,6 Gew.-% NO₃⁻, 1,5 Gew.-% SO₄²⁻ und 85,128 Gew.-% Wasser. Das Produkt ist stabil und zeigt die vergleichbare Reinigungsleistung wie Beispiel 2a

### Beispiel 3a

Produkt nach EP-A-1108679 bestehend aus 5,35 Gew.-% Al³⁺, 16,0 Gew.-% NO₃⁻ und 2,8 Gew.-% S0₄²⁻.

### Beispiel 3 b

Produkt nach EP-A-1108679 bestehend aus 5,35 Gew.-% Al³⁺, 16,0 Gew.-% NO₃⁻, 2,8 Gew.-% SO₄²⁻ und 1 Gew.-% Orangenterpen.

### Beispiel 3 c

Produkt nach EP-A-1108679 bestehend aus 5,35 Gew.-% Al³⁺, 16,0 Gew.-% NO₃⁻, 2,8 Gew.-% SO₄²⁻ und 0,3 Gew.-% einer wässrigen Mischung aus verschiedenen Bakterienkulturen.

### Beispiel 3 d

5 ml der Produkte nach Beispielen 3a, 3b und 3c werden alle 4 Wochen in den Ausguss einer Spüle gegeben. Die Geruchsbelästigung wurde über den Zeitraum der Dosierung verhindert. Nach einem Absetzen der Dosierung tritt die Geruchsentwicklung nach 6 bis 8 Wochen wieder auf.

### Beispiel 3 e

5 ml der Produkte nach Beispielen 3a, 3b und 3c werden wöchentlich in eine Haushebeanlage, an die eine Waschmaschine angeschlossen ist, gegeben. Die Geruchsbelästigung wurde über den Zeitraum der Dosierung verhindert. Nach einem Absetzen der Dosierung tritt die Geruchsentwicklung nach 2 Wochen wieder auf.

## Patentansprüche

1. Verwendung von Nitratsalzen zur Unterdrückung störender biologisch erzeugter Gerüche im Sanitärbereich, insbesondere in Abflüssen, Ausgüssen, Siphons, Hebeanlagen und/oder in Abwasser führenden Bereichen **dadurch gekennzeichnet, dass** die Nitratsalze als Additive zu Reinigungsmitteln, zugegeben werden die Nitratsalze, Aluminiumnitrat, Aluminiumnitratsulfat, Eisennitrat oder Mischungen aus diesen, als Ersatz von organischen Säuren, vorzugsweise Zitronensäure und/oder Essigsäure, und/oder deren Salzen in Reinigungsmitteln eingesetzt werden.

2. Verwendung nach Anspruch 1, **dadurch gekennzeichnet, dass** die Nitratsalze in Form von wässrigen Lösungen eingesetzt werden.

3. Verwendung nach Anspruch 1 oder 2, **dadurch gekennzeichnet, dass** die Konzentration der Nitrat-lonen 0,5 bis 50, bevorzugt 5 bis 40, besonders bevorzugt 10 bis 35 Gew.-% beträgt.

4. Verwendung nach mindestens einem der Ansprüche 1 bis 3, **dadurch gekennzeichnet, dass** 70 bis 90 Gew.-% eines Reinigungsmittels mit 10 bis 30 Gew.-% einer wässrigen Aluminiumnitrat-Lösung gemischt werden.

5. Verwendung von Nitratsalzen nach Anspruch 4, **dadurch gekennzeichnet, dass** die Aluminiumnitrat-Lösung 2 bis 6 Gew.-% Al³⁺ und 20 bis 40, bevorzugt 25 bis 35 Gew.-% NO₃⁻ enthält.

6. Verwendung nach mindestens einem der Ansprüche 1 bis 3, **dadurch gekennzeichnet, dass** 70 bis 90 Gew.-% eines Reinigungsmittels mit 10 bis 30 Gew.-% einer wässrigen Aluminiumnitratsulfat-Lösung gemischt werden.

7. Verwendung nach Anspruch 6, **dadurch gekennzeichnet, dass** die Aluminiumnitratsulfat-Lösung 4 bis 7 Gew.-% Al³⁺, 12 bis 24 Gew.-% NO₃⁻ und 2 bis 3,5 Gew.- % SO₄²⁻ enthält.

8. Verwendung nach mindestens einem der Ansprüche 1 bis 7, **dadurch gekennzeichnet, dass** die nitratsalzhaltigen Lösungen 0,01 bis 5, bevorzugt 0,1 bis 5, besonders bevorzugt 0,5 bis 2 Gew.-% Orangenterpen enthalten.

9. Verwendung nach mindestens einem der Ansprüche 1 bis 7, **dadurch gekennzeichnet, dass** die Mischungen von nitratsalzhaltigen Lösungen mit Reinigungsmitteln 0,01 bis 5, bevorzugt 0,1 bis 5, besonders bevorzugt 0,5 bis 2 Gew.-% Orangenterpen enthalten.

10. Verwendung von Nitratsalzen nach mindestens einem der Ansprüche 1 bis 9, **dadurch gekennzeichnet, dass** die nitratsalzhaltigen Lösungen 0,01 bis 5, bevorzugt 0,05 bis 2, besonders bevorzugt 0,1 bis 1 Gew.-% einer wässrigen Suspension aus Schadstoff abbauenden Bakterien enthalten.

11. Verwendung nach mindestens einem der Ansprüche 1 bis 9, **dadurch gekennzeichnet, dass** die Mischungen von nitratsalzhaltigen Lösungen mit Reinigungsmitteln 0,01 bis 5, bevorzugt 0,05 bis 2, besonders bevorzugt 0,1 bis 1 Gew.-% einer wässrigen Suspension aus Schadstoff abbauenden Bakterien enthalten.

## Claims

1. Use of nitrate salts for suppressing disturbing biologically produced odours in the sanitary field, in particular in waste-pipes, drains, waste traps, pumping systems and/or in waste-water-carrying areas, **characterized in that** the nitrate salts are added as additives to cleaning agents, wherein the nitrate salts, aluminium nitrate, aluminium nitrate sulphate, iron nitrate or mixtures thereof, are used as a replacement for organic acids, preferably citric acid and/or acetic acid, and/or salts thereof in cleaning agents.

2. Use according to claim 1, **characterized in that** the nitrate salts are used in the form of aqueous solutions.

3. Use according to claim 1 or 2, **characterized in that** the concentration of nitrate ions is from 0.5 to 50 wt.%, preferably from 5 to 40 wt.%, particularly preferably from 10 to 35 wt.%.

4. Use according to at least one of claims 1 to 3, **characterized in that** from 70 to 90 wt.% of a cleaning agent are mixed with from 10 to 30 wt.% of an aqueous aluminium nitrate solution.

5. Use of nitrate salts according to claim 4, **characterized in that** the aluminium nitrate solution contains from 2 to 6 wt.% Al³⁺ and from 20 to 40 wt.%, preferably from 25 to 35 wt.%, NO₃⁻.

6. Use according to at least one of claims 1 to 3, **characterized in that** from 70 to 90 wt.% of a cleaning agent are mixed with from 10 to 30 wt.% of an aqueous aluminium nitrate sulphate solution.

7. Use according to claim 6, **characterized in that** the aluminium nitrate sulphate solution contains from 4 to 7 wt.% Al³⁺, from 12 to 24 wt.% NO₃⁻ and from 2 to 3.5 wt.% SO₄²⁻.

8. Use according to at least one of claims 1 to 7, **characterized in that** the nitrate-salt-containing solutions contain from 0.01 to 5 wt.%, preferably from 0.1 to 5 wt.%, particularly preferably from 0.5 to 2 wt.%, orange terpene.

9. Use according to at least one of claims 1 to 7, **characterized in that** the mixtures of nitrate-salt-containing solutions with cleaning agents contain from 0.01 to 5 wt.%, preferably from 0.1 to 5 wt.%, particularly preferably from 0.5 to 2 wt.%, orange terpene.

10. Use of nitrate salts according to at least one of claims 1 to 9, **characterized in that** the nitrate-salt-containing solutions contain from 0.01 to 5 wt.%, preferably from 0.05 to 2 wt.%, particularly preferably from 0.1 to 1 wt.%, of an aqueous suspension of bacteria that degrade harmful substances.

11. Use according to at least one of claims 1 to 9, **characterized in that** the mixtures of nitrate-salt-containing solutions with cleaning agents contain from 0.01 to 5 wt.%, preferably from 0.05 to 2 wt.%, particularly preferably from 0.1 to 1 wt.%, of an aqueous suspension of bacteria that degrade harmful substances.

## Revendications

1. Utilisation de sels nitrates pour supprimer des odeurs gênantes d'origine biologique, produites dans des installations sanitaires, en particulier des voies d'écoulement, des éviers, des siphons, des installations de relevage, et/ou des conduites d'eaux usées, **caractérisée en ce que** les sels nitrates sont ajoutés en tant qu'adjuvants à des nettoyants, étant entendu que l'on introduit dans les nettoyants des sels nitrates, à savoir du nitrate d'aluminium, du nitrate-sulfate d'aluminium, du nitrate de fer ou un mélange de ceux-ci, en tant que produits de remplacement d'acides organiques, de préférence de l'acide citrique et/ou de l'acide acétique, et/ou de sels de tels acides.

2. Utilisation conforme à la revendication 1, **caractérisée en ce que** les sels nitrates sont introduits à l'état de solution aqueuse.

3. Utilisation conforme à la revendication 1 ou 2, **caractérisée en ce que** la concentration des ions nitrate vaut de 0,5 à 50 % en poids, de préférence de 5 à 40 % en poids, et surtout de 10 à 35 % en poids.

4. Utilisation conforme à l'une au moins des revendications 1 à 3, **caractérisée en ce qu'**on a mélangé 70 à 90 % en poids d'un nettoyant avec 10 à 30 % en poids d'une solution aqueuse de nitrate d'aluminium.

5. Utilisation de sels nitrates conforme à la revendication 4, **caractérisée en ce que** la solution de nitrate d'aluminium contient de 2 à 6 % en poids d'ions aluminium Al³⁺ et de 20 à 40 % en poids, de préférence de 25 à 35 % en poids d'ions nitrate NO₃⁻.

6. Utilisation conforme à l'une au moins des revendications 1 à 3, **caractérisée en ce qu'**on a mélangé 70 à 90 % en poids d'un nettoyant avec 10 à 30 % en poids d'une solution aqueuse de nitrate-sulfate d'aluminium.

7. Utilisation conforme à la revendication 6, **caractérisée en ce que** la solution de nitrate-sulfate d'aluminium contient de 4 à 7 % en poids d'ions aluminium Al³⁺, de 12 à 24 % en poids d'ions nitrate NO₃⁻, et de 2 à 3,5 % en poids d'ions sulfate SO₄²⁻.

8. Utilisation conforme à l'une au moins des revendications 1 à 7, **caractérisée en ce que** les solutions contenant des sels nitrates contiennent des terpènes d'orange en une proportion pondérale de 0,01 à 5 %, de préférence de 0,1 à 5 %, et surtout de 0,5 à 2 %.

9. Utilisation conforme à l'une au moins des revendications 1 à 7, **caractérisée en ce que** les mélanges de nettoyant et de solution contenant des sels nitrates contiennent des terpènes d'orange en une proportion pondérale de 0,01 à 5 %, de préférence de 0,1 à 5 %, et surtout de 0,5 à 2 %.

10. Utilisation de sels nitrates, conforme à l'une au moins des revendications 1 à 9, **caractérisée en ce que** les solutions contenant des sels nitrates contiennent une suspension aqueuse de bactéries dégradant les polluants, en une proportion pondérale de 0,01 à 5 %, de préférence de 0,05 à 2 %, et surtout de 0,1 à 1 %.

11. Utilisation de sels nitrates, conforme à l'une au moins des revendications 1 à 9, **caractérisée en ce que** les mélanges de nettoyant et de solution contenant des sels nitrates contiennent une suspension aqueuse de bactéries dégradant les polluants, en une proportion pondérale de 0,01 à 5 %, de préférence de 0,05 à 2 %, et surtout de 0,1 à 1 %.
